# EUROPEAN PATENT APPLICATION

(11) **EP 3 117 856 A1**
(43) Date of publication of application: **18.01.2017**
(21) Application number: 15762140.0
(22) Date of filing: 10.03.2015
(51) Int. Cl.: A61M 5/32

(54) **NEEDLE ASSEMBLY**

(30) Priority: 11.03.2014 JP 2014047160
(71) Applicant: Nipro Corporation, Osaka-shi, Osaka 531-8510 (JP)
(72) Inventor: UCHIDA, Yoshikuni, Tatebayashi-shi Gunma 374-8518 (JP); INAGE, Nobuaki, Tatebayashi-shi Gunma 374-8518 (JP)
(74) Representative: Oxley, Rachel Louise
(86) International application number: PCT/JP2015/056914
(87) International publication number: WO 2015/137298

(57) **Abstract**

A needle assembly (1) includes a cover (5)(7) provided to be movable to a first position where a cannula (2)(3) is exposed and a second position where a distal end projects further than the cannula.

In the cover, circumferential-direction locking sections (25)(43) or front-back-direction grooves (24)(42) continuing to the circumferential-direction locking sections or engaging protrusions (5a)(33a) movable along the circumferential-direction locking sections and the front-back-direction grooves are provided.

In a state in which the cover is located in the first position and the engaging protrusions are housed in the circumferential-direction locking sections, movement in a front-back direction of the cover is blocked. When the engaging protrusions move along the circumferential-direction locking sections and move to the front-back-direction grooves, the engaging protrusions become movable along the front-back-direction grooves and allow the movement to the second position of the cover.

Projecting sections to the outside are reduced as much as possible and movement of the cover due to a malfunction is prevented.

## Description

### Technical Field

The present invention relates to a needle assembly and, more particularly, to a needle assembly including a cannula in which a puncture section is formed, a hub that holds the cannula, and a cover provided to be movable to a first position where the cannula is exposed with respect to the hub and a second position where a distal end projects further than the cannula.

### Background Art

In order to prevent erroneous puncture after use, there has been known a needle assembly including a cannula in which a puncture section is formed, a hub that holds the cannula, and a cover provided to be movable to a first position where the cannula is exposed with respect to the hub and a second position where a distal end projects further than the cannula.

As such a needle assembly, there has been known a needle assembly including a front cannula in which a puncture section is formed at a distal end, a hub that holds the front cannula, a front cover provided to be movable to a retracted position where the front cannula is exposed with respect to the hub and an advanced position where a distal end projects further to the front than the front cannula, and urging means for urging the front cover toward the advanced position (Patent Document 1).

In such a needle assembly, an operation lever for performing operation and an engaging protrusion for holding the front cover in the retracted position are exposed to the outside. A user operates the lever to thereby locate the front cover in the advanced position.

### Prior Art Document

### Patent Document

Patent Document 1: Japanese Patent Laid-Open No. 2007-151752

### Summary of Invention

### Problems to be Solved by the Invention

However, for example, when the needle assembly is used for dental treatment, since the operation lever projects to the outside, it is likely that a patient may be injured by the lever. It is also likely that the front cover erroneously projects to the advanced position because of erroneous operation by a user who holds the assembly in an oral cavity.

In view of such problems, the present invention provides a needle assembly capable of reducing projecting objects to the outside as much as possible and preventing a malfunction.

### Means for Solving the Problems

That is, a needle assembly described in claim 1 is a needle assembly including: a cannula in which a puncture section is formed; a hub that holds the cannula; and a cover provided to be movable to a first position where the cannula is exposed with respect to the hub and a second position where a distal end projects further than the cannula, characterized in that
in the cover, a circumferential-direction locking section formed in a circumferential direction and a front-back-direction groove formed in a front-back direction to continue to the circumferential-direction locking section or an engaging protrusion movable along the circumferential-direction locking section and the front-back-direction groove are provided,
the movement of the cover in the front-back direction is blocked in a state in which the cover is located in the first position and the engaging protrusion is housed in the circumferential-direction locking section, and
when the engaging protrusion moves along the circumferential-direction locking section and moves to the front-back-direction groove, the engaging protrusion becomes movable along the front-back-direction groove to allow the movement of the cover to the second position.

### Advantageous Effects of Invention

According to the invention of claim 1, in the state in which the cover is located in the first position, since the engaging protrusion is housed in the circumferential-direction locking section, it is possible to prevent injury to the patient by the operation lever projecting to the outside described in Patent Document 1.

To move the cover to the second position, the engaging protrusion needs to be moved along the circumferential-direction locking section and moved to the front-back-direction groove. Therefore, it is possible to prevent the projection of the cover due to a malfunction even if operation in a narrow place is performed.

### Brief Description of Drawings

[Figure 1] Figure 1 is an exterior view of a state of use of a needle assembly according to an embodiment; (a) and (b) respectively show figures of the needle assembly viewed from different angles.
[Figure 2] Figure 2 is an exterior view of a state after use of the needle assembly.
[Figure 3] Figure 3 is sectional views in a III-III(a) part and a III-III(b) part in Figure 1.
[Figure 4] Figure 4 is a sectional view in a IV-IV part of Figure 2.
[Figure 5] Figure 5 is a sectional view in a V-V part in Figure 1.
[Figure 6] Figure 6 is a sectional view in a VI-VI part in Figure 2.
[Figure 7] Figure 7 shows a needle assembly according to a second embodiment; (a) shows a sectional view of a state of use and (b) shows a figure of the needle assembly viewed from a front cannula side in the state of use.
[Figure 8] Figure 8 is a sectional view of a needle assembly according to a third embodiment; (a) shows a state of use and (b) shows a state after use.

### Mode for Carrying out the Invention

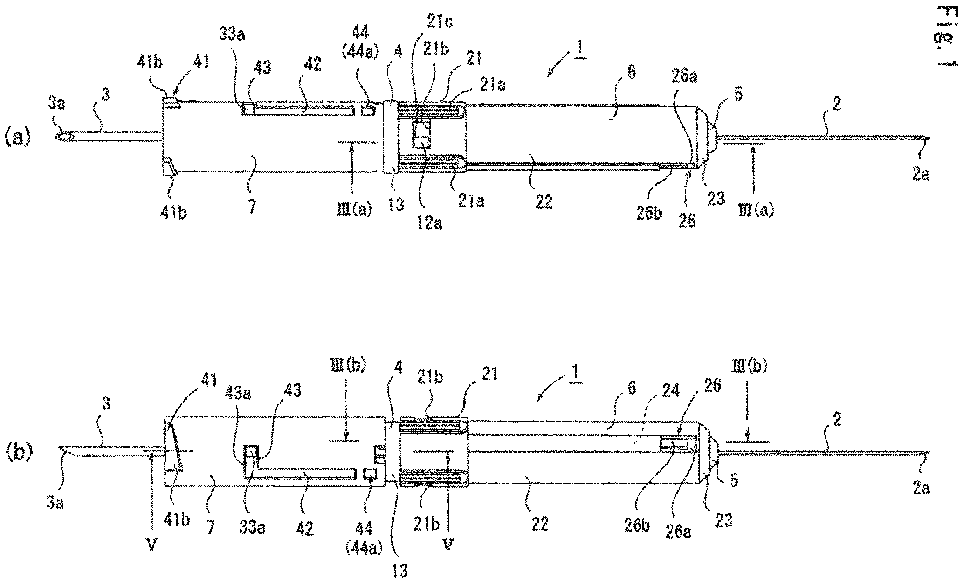

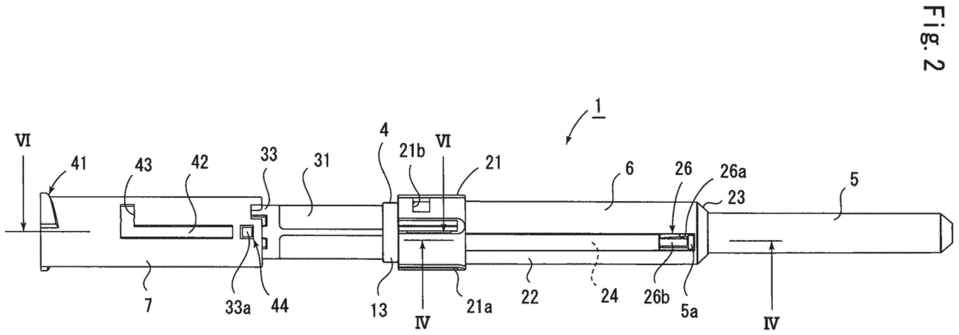

Embodiments shown in the figures are explained below. A needle assembly (1) shown in Figures 1 and 2 is mounted on not-shown liquid supplying means such as a syringe, in which an anesthetic is stored, and used in dental treatment. Figure 1 shows a state of use of the assembly (1). Figure 2 shows a state after use of the assembly (1).

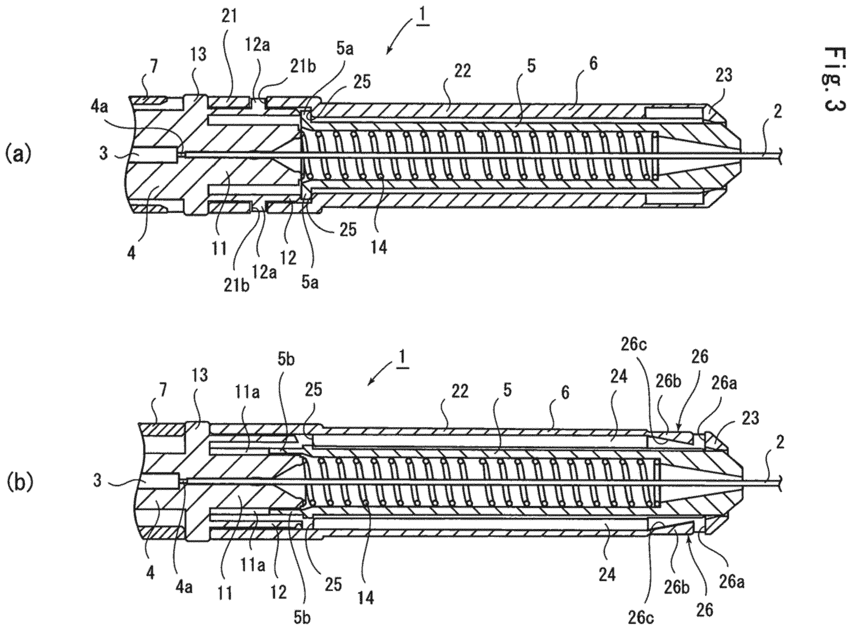

Parts (a) and (b) of Figure 1 respectively show the assembly (1) in the state of use viewed from different angles. Figure 3 to Figure 6 respectively show sectional views of sections in Figures 1 and 2. In the figures, the right shown in the figures is referred to as front or distal end side and the left shown in the figures is referred to as back or rear end side.

The needle assembly (1) includes a front cannula (2) projecting to the front, a back cannula (3) projecting backward, a hub (4) that holds the front cannula (2) and the back cannula (3), a front cover (5) (see Figure 2) provided to be capable of advancing and retracting in the front of the hub (4), a cover guide (6) provided in the hub (4) to cover the front cover (5), and a back cover (7) provided to be capable of advancing and retracing in the back of the hub (4).

Further, in the state of use of the needle assembly (1) shown in Figure 1, the assembly (1) is in a puncturable state in which the front cover (5) is located in a retracted position serving as a first position, the back cover (7) is located in an advanced position serving as the second position, and the front cannula (2) and the back cannula (3) are exposed to the outside.

On the other hand, in the state after use of the needle assembly (1) shown in Figure 2, the assembly (1) is in a state in which the front cover (5) is located in the advanced position serving as a second position, the back cover (7) is located in the retracted position serving as the first position, and the front cannula (2) and the back cannula (3) are housed on the insides of the front cover (5) and the back cover (7), and erroneous puncture is prevented.

The front cannula (2) and the back cannula (3) are respectively configured by needle tubes made of stainless steel or the like. Puncture sections (2a) and (3a) are respectively formed at the distal end of the front cannula (2) and the rear end of the back cannula (3).

The hub (4) is a substantially columnar member. A through-hole (4a) piercing through the hub (4) to the front and back is drilled on the inside of the hub (4) (see Figure 3). The front cannula (2) and the back cannula (3) are bonded and held in the through-hole (4a). In the through-hole (4a), a slight gap is formed between the rear end portion of the front cannula (2) and the distal end portion of the back cannula (3).

The diameter of the front cannula (2) in the needle assembly (1) for dental use is set to as small a diameter as possible in order to reduce puncture pain. The total length of the cannula (2) is set as long as possible such that the assembly (1) can be punctured in any position in an oral cavity.

Therefore, an inner passage of the cannula (2) is small in diameter and channel resistance is large. Therefore, the liquid supplying means has to feed the anesthetic with a high supply pressure.

On the other hand, the back cannula (3) is provided for the purpose of piercing through a lid section made of resin provided at the distal end portion of the liquid supplying means. Therefore, it is unnecessary to set the diameter of the back cannula (3) to a small diameter. Chanel resistance can be reduced.

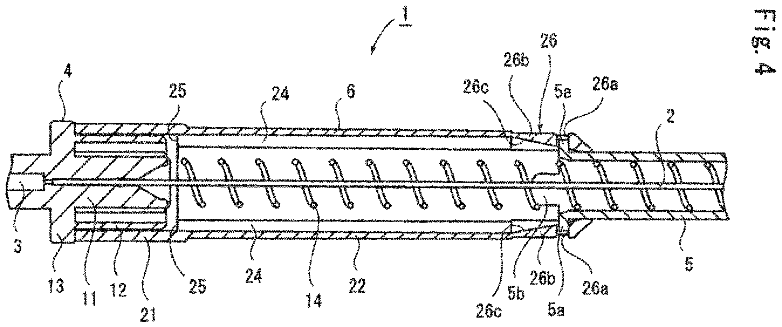

The configurations and the operations of the front cover (5) and the cover guide (6) are explained below with reference to Figure 3 and Figure 4. Figure 3 shows a state of use in which the cover (5) is located in the retracted position. Figure 4 shows a state after use in which the cover (5) moves to the advanced position.

At the distal end of the hub (4), a columnar front-side inner cylinder section (11), which holds the front cannula (2), and a cylindrical front-side outer cylinder section (12), which surrounds the inner cylinder section (11), are formed. A substantially disc-like flange (13) is formed in the back of the inner section (11) and the outer section (12).

The distal ends of the inner section (11) and the outer section (12) are formed in substantially the same positions. A required gap is formed between the inner section (11) and the outer section (12).

The rear end portion of a spring (14) functioning as urging means elastically installed between the distal end portion of the front-side inner cylinder section (11) and the front cover (5) is elastically in contact with the distal end of the inner section (11). As shown in Figure 3(b), key grooves (11a) are formed toward the front-back direction in positions opposed to each other across the front cannula (2) on the side surface of the inner cylinder section (11).

The front-side outer cylinder section (12) is formed smaller in diameter than the diameter of the flange (13). On the outer circumferential surface of the outer cylinder section (12), cover-guide holding protrusions (12a) project outward in positions opposed to each other across the front cannula (2).

The front cover (5) is formed in a substantially cylindrical shape. When the front cover (5) is located in the advanced position as shown in Figure 2, the distal end of the front cover (5) projects further to the front than the puncture section (2a) of the front cannula (2) and covers the section (2a).

When the front cover (5) is located in the retracted position as shown in Figure 1, the front cover (5) also projects slightly further than the cover guide (6). The distal end portion of the cover (5) is formed in a taper shape. Consequently, a patient is not injured when the needle assembly (1) is used in an oral cavity.

The distal end portion of the inner surface of the front cover (5) is also formed in a taper shape. The spring (14) is elastically in contact with the rear end portion of the taper shape from the back. Consequently, the cover (5) is always urged to the front by the spring (14).

At the rear end portion of the front cover (5), a pair of front-side engaging protrusions (5a) projecting to the outer circumference side and a pair of keys (5b) projecting to the back are respectively formed in positions opposed to each other across the front cannula (2). The protrusions (5a) and the keys (5b) are formed in positions biased a predetermined angle centering on the axial direction of the cannula (2).

The keys (5b) engage with the key grooves (11a) of the hub (4) to configure rotation preventing means for the front cover (5). As shown in Figure 3(b), in a state in which the front cover (5) is located in the retracted position, the keys (5b) fit in the grooves (11a) to thereby prevent the rotation of the front cover (5) and the hub (4).

The cover guide (6) is formed as a substantially cylindrical member. A large diameter section (21), which fits with the front-side outer cylinder section (12) of the hub (4), an intermediate diameter section (22), which houses the front cover (5), and a small diameter section (23) formed at the distal end of the guide (6) are formed in order from the rear end of the guide (6).

The large diameter section (21) fits with the outer cylinder section (12) to be capable of rotating in the circumferential direction. A plurality of protrusions (21a) for operation to be held by a user in treatment are formed on the outer circumferential surface of the large diameter section (21) (see Figure 1 and Figure 2).

In the large diameter section (21), cover-guide holding grooves (21b), which engage with the cover-guide holding protrusions (12a) formed in the hub (4), are formed toward the circumferential direction of the section (21).

When the cover-guide holding protrusions (12a) are housed in the cover-guide holding grooves (21b), the movement in the front-back direction of the cover guide (6) with respect to the hub (4) is blocked and, on the other hand, the rotation of the guide (6) in the circumferential direction is allowed.

Specifically, in the state of use shown in Figure 1, as shown in Figure 1(a), the protrusions (12a) are located on the lower side shown in the figure of the grooves (21b). It is possible to rotate the cover guide (6) clockwise from this state.

Very small protrusions (21c) are formed in the cover-guide holding grooves (21b). When the user rotates the cover guide (6), the user moves the cover-guide holding protrusions (12a) to move over the protrusions (21c).

On the inner surface of the cover guide (6), front-side front-back-direction grooves (24) formed toward the front-back direction and front-side circumferential-direction locking sections (25) formed continuously in the circumferential direction from the rear end portions of the grooves (24) are formed. Front-cover-retraction blocking sections (26) for blocking retraction of the front cover (5) are provided in the front of the grooves (24). Pairs of the grooves (24), the locking sections (25), and the blocking sections (26) are respectively provided across the front cannula (2).

The front-side front-back-direction grooves (24) are formed at depth for enabling the front-side engaging protrusions (5a) of the front cover (5) to pass through the grooves (24). The grooves (24) are formed not to pierce through the cover guide (6) to the outer surface.

By forming the grooves (24) not to pierce through the cover guide (6) to the outer surface, when the guide (6) is led into the oral cavity of the patient, the patient is prevented from being injured by protrusions or edges formed by the grooves that pierce through the guide (6).

The front-side circumferential-direction locking sections (25) are configured by a step formed in the boundary between the large diameter section (21) and the intermediate diameter section (22) of the cover guide (6). As shown in Figure 3(b), the locking sections (25) are formed to continue to the rear end portions of the front-side front-back-direction grooves (24).

Gaps in a degree in which the front-side engaging protrusions (5a) can be located are formed between the locking sections (25) and the front-side outer cylinder section (12) of the hub (4).

The inner diameter of the small diameter section (23) is set to a diameter for disabling the front-side engaging protrusions (5a) of the front cover (5) to pass through the section (23). When the front cover (5) is located in the advanced position as shown in Figure 4, the front-side engaging protrusions (5a) come into contact with a surface in the back of the small diameter section (23) and block further advance of the front cover (5).

The outer surface of the section (23) is formed in a taper shape. The taper-shaped portion of the front cover (5) projects slightly further to the front than the small diameter section (23) in the retracted position. An integral taper shape is formed by the section (23) and the distal end portion of the cover (5) to prevent the patient from being injured.

The front-cover-retraction blocking sections (26) are located between the small diameter section (23) and the front-side front-back-direction grooves (24) and configured from through-holes (26a) formed in a substantially rectangular shape and retraction blocking protrusions (26b) projecting to the insides of the holes (26a).

The surfaces in the front in the holes (26a) configure the surface at the rear end of the small diameter section (23). As explained above, the front-side engaging protrusions (5a) of the front cover (5) come into contact with the surface from the back and block the advance of the cover (5).

The retraction blocking protrusions (26b) are formed to project toward the front from the surfaces in the back of the holes (26a). As shown in Figure 4, the engaging protrusions (5a) are housed between the distal end portions of the blocking protrusions (26b) and the surfaces in the front of the holes (26a).

The retraction blocking protrusions (26b) are elastically deformed starting from a base section on the rear end side of the retraction blocking protrusions (26b). Inclined surfaces (26c) inclining from the back toward the front are formed on the inner surfaces of the retraction blocking protrusions (26b).

The front-side engaging protrusions (5a) come into contact with the inclined surfaces (26c). While the engaging protrusions (5a) pass, the blocking protrusions (26b) move outward.

On the other hand, the outer surface side of the blocking protrusions (26b) is located flush with the outer surface of the cover guide (6) or further on the inner surface side than the outer surface to prevent the patient from being injured when the needle assembly (1) is operated in the oral cavity.

Concerning the configuration explained above, an operation in locating the front cover (5) in the advanced position from the retracted position is explained below.

First, in the state of use, when the front cover (5) is located in the retracted position, as shown in Figure 3, the front-side engaging protrusions (5a) formed at the rear end portion of the cover (5) are in contact with, from the back, the front-side circumferential-direction locking sections (25) formed in the cover guide (6). The advance of the front cover (5) is blocked irrespective of an urging force of the spring (14).

In this state, when the cover guide (6) is rotated clockwise from the state of Figure 1, the engaging protrusions (5a) relatively move along the locking sections (25).

At this point, since the keys (5b) of the cover (5) located in the retracted position engage with the key grooves (11a) of the hub (4), the rotation of the cover (5) is blocked. The engaging protrusions (5a) of the cover (5) move relatively to the locking sections (25).

A rotation amount of the cover guide (6) is specified by the length in the circumferential direction of the cover-guide holding grooves (21b) formed in the guide (6), that is, a movement amount of the cover-guide holding protrusions (12a) formed in the hub (4).

When the holding protrusions (12a) move from the state shown in Figure 1 to the other end portions in the holding grooves (21b), the front-side front-back-direction grooves (24) of the cover guide (6) move to positions overlapping the front-side engaging protrusions (5a).

When the grooves (24) move to the positions of the engaging protrusions (5a) in this way, the protrusions (5a) are capable of advancing along the grooves (24). The front cover (5) advances to the advanced position with the urging force of the spring (14).

When the front cover (5) advances with the urging force of the spring (14), the front-side engaging protrusions (5a) advance along the front-side front-back-direction grooves (24) and come into contact with, from the back, the inclined surface (26c) formed on the inner surfaces of the retraction blocking protrusions (26b) in the front-cover-retraction blocking sections (26).

Then, the blocking protrusions (26b) are pressed outward by the engaging protrusions (5a). Consequently, the base sections on the rear end portion side of the blocking protrusions (26b) are elastically deformed to move outward and allow passage of the engaging protrusions (5a).

The engaging protrusions (5a) come into contact with the surfaces in the front of the through-holes (26a), which have passed the blocking protrusions (26b), and are located in the advanced position shown in Figure 4. Consequently, further advance of the front cover (5) is blocked.

On the other hand, when the engaging protrusions (5a) pass the blocking protrusions (26b), the blocking protrusions (26b) return from the elastic deformation and move inward again. The distal end portions of the blocking protrusions (26b) become capable of coming into contact with the passed engaging protrusions (5a) from the back. Therefore, the retraction of the front cover (5) is blocked.

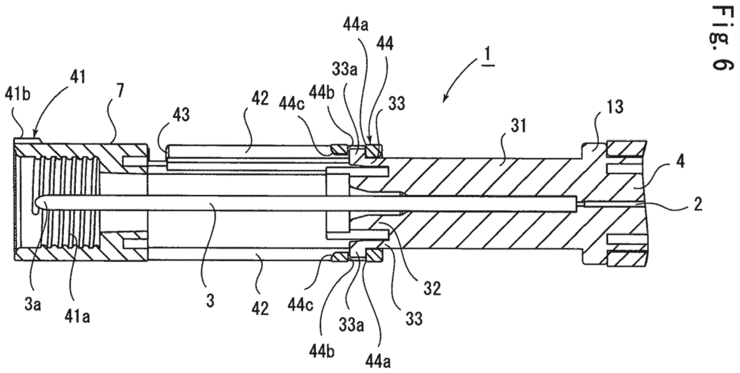

The configuration and the operation of the back cover (7) are explained with reference to Figure 4 and Figure 6.

In the back of the flange (13) in the hub (4), a back-cannula holding section (31) formed in a substantially cross shape in section, which holds the back cannula (3), a columnar back-side inner cylinder section (32) projecting to the back from the holding section (31), and a cylindrical back-side outer cylinder section (33) that surrounds the inner section (32) are formed.

The through-hole (4a) is formed in the center of the holding section (31) and the inner section (32). The rear end portions of the inner section (32) and the outer section (33) are formed in substantially the same positions. A required gap is formed between the inner section (32) and the outer section (33).

In the back-side outer cylinder section (33), a pair of back-side engaging protrusions (33a) is provided in positions opposed to each other across the back cannula (3). The pair of engaging protrusions (33a) is formed to project to the outer circumference side of the outer cylinder section (33).

The back-side engaging protrusions (33a) are provided further at rear end portions of an arm section projecting to the back from the rear end portion of the back-cannula holding section (31). When the arm section is elastically deformed, the engaging protrusions (33a) move to the inner circumference side.

The back cover (7) is formed as a cylindrical member. The cover (7) slides in the front-back direction along the back-cannula holding section (31) and the back-side outer cylinder section (33) of the hub (4) and is capable of rotating in the circumferential direction. When the cover (7) is located in the retracted position as shown in Figure 2, the rear end of the cover (7) projects further to the back than the puncture section (3a) of the back cannula (3) and covers the section (3a).

At the rear end portion of the back cover (7), a coupling section (41) coupled to a coupled section provided in the not--shown liquid supplying means is formed. The coupling section (41) is configured from a female screw section (41a) formed on the inner surface and a lock section (41b) formed on the outer surface. On the other hand, in the coupled section of the not-shown liquid supplying means, a male screw section, which screws in the female screw section (41a), and a lock section, which engages with the lock section (41b), are provided.

In order to couple the coupled section of the liquid supplying means to the coupling section (41), the liquid supplying means is rotated. Consequently, the male screw section screws with the female screw section (41a). The lock section (41b) engages with the lock section.

On the other hand, to separate the coupled section of the liquid supplying means from the coupling section (41) in order to separate the liquid supplying means from the needle assembly (1), the liquid supplying means only has to be rotated in the opposite direction to separate the male screw section from the female screw section (41a) of the back cover (7).

Note that such a coupling section (41) is publicly known in the past. The section (41) in this embodiment may be coupling sections corresponding to liquid supplying means including coupled sections having other shapes.

In the back cover (7), back-side front-back-direction grooves (42) formed toward the front-back direction, back-side circumferential-direction locking sections (43) formed continuously to the rear end portions of the grooves (42), and back-cover-advance blocking sections (44) provided in the front of the grooves (42) are provided. Pairs of the grooves (42), the locking sections (43), and the blocking sections (44) are respectively provided in positions across the back cannula (3).

The back-side engaging protrusions (33a) formed in the hub (4) move to the back-side front-back-direction grooves (42). Unlike the front-side front-back-direction grooves (24) formed in the cover guide (6), the back-side front-back-direction grooves (42) may pierce through the back cover (7) from the inner surface to the outer surface.

As shown in Figure 1(b), the back-side circumferential-direction locking sections (43) are formed continuously in the circumferential direction from the rear end portions of the grooves (42) and formed in a groove shape to match the width of the engaging protrusions (33a).

In Figure 1(b), the locking sections (43) are formed upward shown in the figure from the rear end portions of the grooves (42). When the back cover (7) is rotated counterclockwise, the engaging protrusions (33a) relatively move along the locking sections (43).

Further, in the locking sections (43), protrusions (43a) are formed in positions adjacent to the back-side engaging protrusions (33a). The back cover (7) is prevented from unintentionally rotating.

The back-cover-advance blocking sections (44) are configured from through-holes (44a) formed in positions spaced apart from the grooves (42) to the front by a predetermined distance and piercing through the back cover (7) and advance blocking sections (44b) formed between the through-holes and the grooves (42).

The through-holes (44a) are formed in a shape for allowing the back-side engaging protrusions (33a) to fit in the holes (44a) substantially without a gap. The engaging protrusions (33a) come into contact with the surfaces in the front of the holes (44a) from the back to block further retraction of the back cover (7).

With the through-holes (44a), since the engaging protrusions (33a) are housed in the holes (44a), the protrusions (33a) can be visually recognized from the outside (see Figure 2).

The advance blocking sections (44b) configure the surfaces in the back of the holes (44a). The engaging protrusions (33a) come into contact with the advance blocking sections (44b) from the front to block the advance of the back cover (7).

Further, on the inner surfaces of the advance blocking sections (44b), inclined surfaces (44c) projecting to the inner surface side are formed from the back to the front. The engaging protrusions (33a) of the hub (4) come into contact with the inclined surfaces (44c) when the back cover (7) retracts.

Concerning the configuration explained above, an operation in locating the back cover (7) from the advanced position to the retracted position is explained.

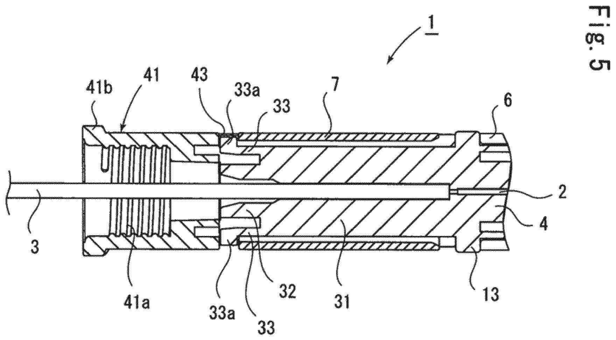

In the state of use shown in Figure 1, in a state in which the back cover (7) is located in the advanced position, as shown in Figure 5, the back-side engaging protrusions (33a) are housed in the back-side circumferential-direction locking sections (43). The retraction of the cover (7) is blocked.

In a state in which engaging protrusions (33a) are housed in the locking sections (43) in this way, when the cover (7) is rotated counterclockwise from the state shown in Figure 1, the engaging protrusions (33a) relatively move to the rear end portions of the grooves (42) along the locking sections (43).

When the user retracts the back cover (7) from that state, the engaging protrusions (33a) advance relatively to the grooves (42). The protrusions (33a) come into contact with the inclined surface (44c) of the blocking sections (44) from the back.

Then, the engaging protrusions (33a) are elastically deformed from the base sections on the distal end side thereof, move inward toward the inner cylinder section (32), and pass the inclined surfaces (44c). After passing the surfaces (44c), the protrusions (33a) move outward again and are housed in the through-holes (44a).

When the engaging protrusions (33a) are housed in the through-holes (44a), further retraction of the back cover (7) is blocked by the surfaces in the front of the holes (44a). The cover (7) is located in the retracted position. Repeated advance of the cover (7) is blocked by the advance blocking sections (44b).

A method of using the needle assembly (1) having the configuration explained above is explained below.

First, the needle assembly (1) is conveyed in a state in which the assembly (1) is housed on the inside of a not-shown casing. The casing is configured from a front side casing that covers the cover guide (6) and the front cannula (2) and a back side casing that covers the back cover (7) and the back cannula (3).

The user removes the back side casing of the casing and exposes the back cover (7) and the back cannula (3). In this case, the needle assembly (1) is in the state of use shown in Figure 1. The cover (7) is held in the advanced position.

In this state, the user couples the coupled section of the liquid supplying means to the coupling section (41) formed in the cover (7). Consequently, the back cannula (3) pierces through the lid section of the liquid supplying means. The anesthetic can be supplied from the liquid supplying means to the cannula (3).

Subsequently, the user removes the front side casing of the casing and exposes the cover guide (6) and the front cannula (2). At this point, the front cover (5) is held in the retracted position.

The user grips the flange (13) of the hub (4) and the protrusions (21a) for operation of the cover guide (6), punctures the front cannula (2) into the oral cavity of the patient, and operates the liquid supplying means to administer the anesthetic.

In that case, since protrusions are not formed as much as possible on a portion led into the oral cavity of the patient, that is, the outer circumferential surface of the cover guide (6), it is possible to prevent the patient from being injured by the protrusions.

The front-side engaging protrusions (5a) of the front cover (5) are not exposed to the outside. Therefore, the user gripping the needle assembly (1) is prevented from erroneously touching the engaging protrusions (5a) to cause the front cover (5) to malfunction.

When the user finishes the administration of the anesthetic to the patient, the user rotates the cover guide (6) clockwise with respect to the hub (4). Consequently, the front cover (5) moves from the retracted position to the advanced position and changes from the state of use to the state after use.

When rotating the cover guide (6), the user has to cause, with the cover-guide holding protrusions (12a), the guide (6) to move over the protrusions (21c) formed in the cover-guide holding grooves (21b). The guide (6) is prevented from unintentionally rotating to cause the front cover (5) to malfunction before the guide (6) moves over the protrusions (21c).

Since the front cover (5) changes to the state after use, the distal end of the cover (5) projects further to the front than the puncture section (2a) of the front cannula (2). Therefore, erroneous puncture by the cannula (2) is prevented.

When the front cover (5) is located in the advanced position, the front-side engaging protrusions (5a) are housed in the front-cover-retraction blocking sections (26) provided in the front of the front-side front-back-direction grooves (24). The cover (5) is located in the advanced position and repeated retraction is blocked.

When the front cover (5) is located in the advanced position in this way, subsequently, in a state in which the liquid supplying means is kept connected, the user retracts the back cover (7) from the advanced position to the retracted position and changes the cover (7) from the state of use to the state after use.

Specifically, the user rotates the cover (7) counterclockwise from the state shown in Figure 1 and, when the back-side engaging protrusions (33a) are located at the rear end portions of the back-side front-back-direction grooves (42), moves the cover (7) to the back.

When rotating the back cover (7), the user has to cause, with the engaging protrusions (33a), the cover (7) to move over the protrusions (21c) formed in the back-side circumferential-direction locking sections (43). The cover (7) is prevented from unintentionally rotating to retract before the cover (7) moves over the protrusions (21c) .

When the cover (7) is located in the retracted position, the engaging protrusions (33a) are housed in the through-holes (44a). By visually recognizing the engaging protrusions (33a) from the outside, the user can confirm that the back cover (7) is surely located in the retracted position.

When the back cover (7) is located in the retracted position in this way, the user rotates the liquid supplying means coupled to the coupling section (41) of the cover (7) counterclockwise. Consequently, the user can separate the lock section and the male screw section of the coupled section from the lock section (41b) and the female screw section (41a) of the coupling section (41) .

When the liquid supplying means is separated in this way, since the cover (7) is already located in the retracted position and is in the state after use shown in Figure 2, the back cannula (3) is housed in the cover (7). It is possible to prevent subsequent erroneous puncture by the cannula (3).

Thereafter, the needle assembly (1) in this embodiment is discarded. However, since the front cover (5) is maintained in the advanced position and the back cover (7) is maintained in the retracted position as explained above, it is also possible to prevent an accident in which the front cannula (2) and the back cannula (3) are exposed from the discarded assembly (1).

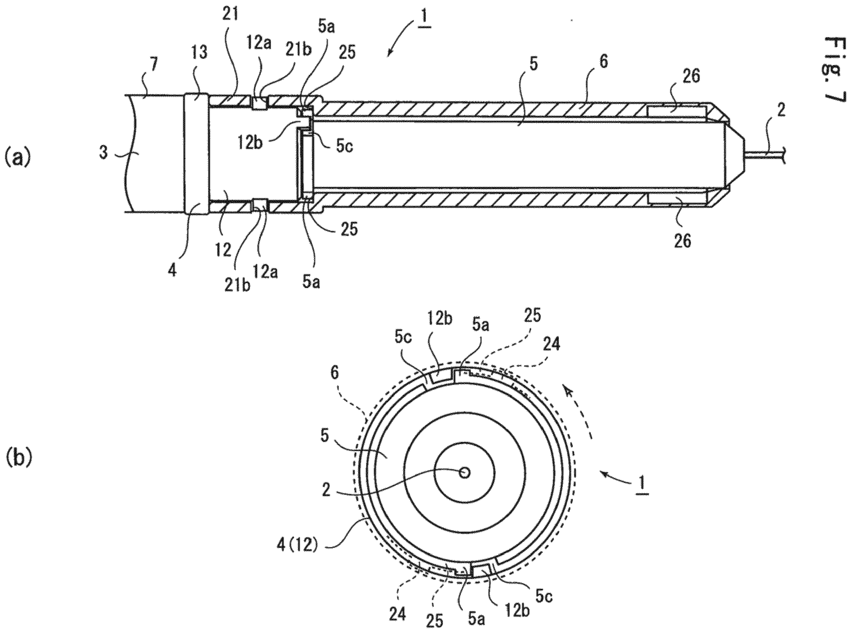

Figure 7 shows a partial sectional view (a) on the front cover (5) side of the needle assembly (1) according to the second embodiment and a view (b) of the assembly (1) viewed from the distal end side. Specifically, Figure 7 is a figure for explaining another embodiment concerning rotation preventing means for blocking the rotation of the hub (4) and the cover (5). In this embodiment, explanation is omitted concerning elements common to the elements in the first embodiment and the same reference numerals and signs are used for the elements.

As the cover guide (6) in this embodiment, a cover guide same as the cover guide (6) in the first embodiment can be used. The front-side front-back-direction grooves (24) and the front-side circumferential-direction locking sections (25) are formed in the guide (6).

The rotation preventing means of the front cover (5) in this embodiment is configured from engaging concave sections (5c) formed at the rear end portion of the cover (5) and formed in positions adjacent to the front-side engaging protrusions (5a) and engaging convex sections (12b) formed at the distal end portion of the front-side outer cylinder section (12) of the hub (4) and engaging with the engaging concave sections (5c).

Concerning the needle assembly (1) having the configuration explained above, an operation in locating the front cover (5) in the advanced position from the retracted position is explained below.

As in the first embodiment, in a state in which the cover (5) is located in the retracted position, the front-side engaging protrusions (5a) formed at the rear end portion of the cover (5) come into contact with, from the back, the front-side circumferential-direction locking sections (25) formed in the cover guide (6) and block the advance of the cover (5) resisting the urging force of the spring (14).

In this state, when the guide (6) is rotated counterclockwise from a state shown in Figure 7(b) with respect to the hub, the cover (5) located on the inside of the guide (6) is also about to rotate with friction or the like.

However, the engaging protrusions (5a) are in contact with the engaging convex sections (12b) of the hub (4) located in the engaging concave sections (5c). Consequently, the rotation of the cover (5) is prevented.

As a result, the cover guide (6) and the front cover (5) relatively rotate. The front-side engaging protrusions (5a) relatively move along the front-side circumferential-direction locking sections (25) and move to positions overlapping the front-side front-back-direction grooves (24).

When the grooves (24) move to the positions of the engaging protrusions (5a), the protrusions (5a) are capable of advancing along the grooves (24). The front cover (5) advances to the advanced position with the urging force of the spring (14).

When the front cover (5) is located in the advanced position, as in the first embodiment, the engaging protrusions (5a) are located in the front-cover-retraction blocking sections (26) formed on the distal end side of the grooves (24). The advance and the retraction of the cover (5) are blocked.

Note that, as the rotation preventing means for preventing the rotation of the hub (4) and the front cover (5) described in the first and second embodiments, besides, it is also possible to couple and fix the hub (4) and the cover (5) with the spring (14) and prevent the rotation of the hub (4) and the cover (5) with the spring (14). Further, it is also conceivable to integrally form the hub (4), the cover (5), and the spring (14) with resin.

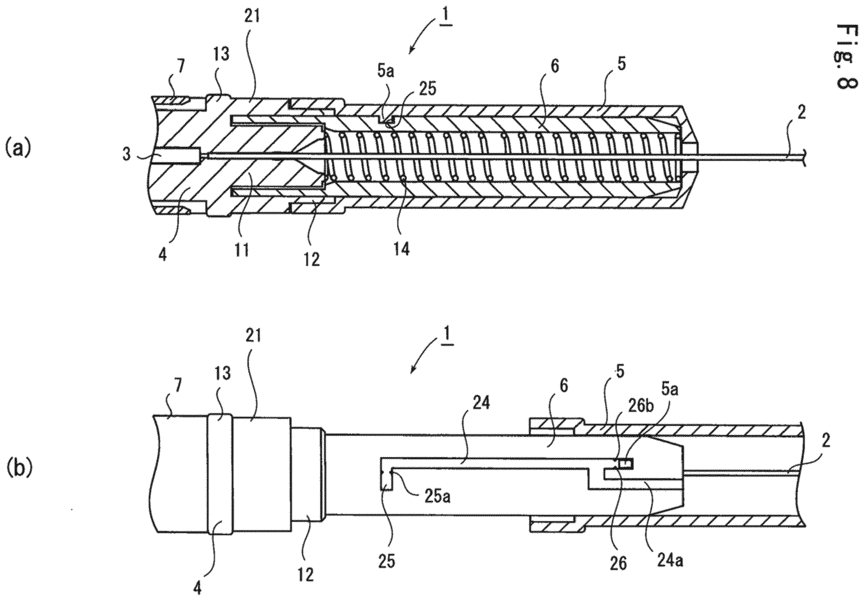

Figure 8 shows the needle assembly (1) according to a third embodiment and (a) shows a state in which the front cover (5) is located in the retracted position and (b) shows a state in which the cover (5) is located in the advanced position.

The needle assembly (1) in this embodiment has a configuration in which the cover (5) is located on the outer circumference side and the cover guide (6) is housed on the inside of the cover (5).

The rear end portion of the cover guide (6) is inserted between the front-side inner cylinder section (11) and the front-side outer cylinder section (12) of the hub (4) and is coupled and fixed not to be capable of rotating with respect to the hub (4).

The spring (14) is housed on the inside of the cover guide (6). A distal end portion opening of the cover guide (6) is set to a diameter same as the diameter of the spring (14). Consequently, the spring (14) projects further to the front than the opening section.

In this embodiment, on the outer circumferential surface of the cover guide (6), as shown in (b), the front-side front-back-direction grooves (24), the front-side circumferential-direction locking sections (25), and the front-cover-retraction blocking sections (26) are formed.

Specifically, the grooves (24) are formed on the outer circumferential surface of the cover guide (6) toward the front-back direction and configured to house the front-side engaging protrusions (5a) formed on the inner circumferential surface of the front cover (5).

The locking sections (25) are grooves formed continuously in the circumferential direction from the rear end portions of the grooves (24). Protrusions (25a) for blocking the movement of the engaging protrusions (5a) are formed halfway in the locking sections (25).

The blocking sections (26) include retraction blocking protrusions (26b) formed near the distal end portions of the grooves (24). When the engaging protrusions (5a) are located at the distal ends of the grooves (24), the blocking sections (26) engage with the engaging protrusions (5a) from the back to block the retraction of the cover (5).

Further, grooves (24a) for assembly branching from the front-side front-back-direction grooves (24) are formed in positions adjacent to the back of the blocking sections (26) in this embodiment.

The grooves (24a) for assembly are formed to the distal end of the guide (6). When the needle assembly (1) is assembled, the engaging protrusions (5a) of the front cover (5) are intruded into the grooves (24a) for assembly. The engaging protrusions (5a) are led into the locking sections (25) via the front-side front-back-direction grooves (24).

The front cover (5) is formed to have a cylindrical shape and surround the cover guide (6) and the front-side outer cylinder section (12) of the hub (4) and provided to be capable of rotating relatively to the hub (4) and the cover guide (6).

The distal end portion of the front cover (5) is located further in the front than the distal end portion of the guide (6). The opening section of the cover (5) is formed smaller in diameter than the distal end opening section of the guide (6).

The spring (14) is elastically in contact with, from the back, the rear side of the distal end opening section of the front cover (5) and always urges the cover (5) toward the advanced position.

On the inner circumferential surface of the front cover (5), the front-side engaging protrusions (5a), which engage with the front-side front-back-direction grooves (24) and the front-side circumferential-direction locking sections (25) formed on the outer circumference of the cover guide (6), are provided.

In a state in which the front-side engaging protrusions (5a) are housed in the locking sections (25), the front cover (5) is regulated from moving to the front and held in the retracted position. In a range in which the protrusions (5a) move within a range in which the locking sections 25 are formed, the cover (5) and the guide (6) are capable of relatively rotating.

Concerning the needle assembly (1) having the configuration explained above, an operation in locating the front cover (5) in the advanced position from the retracted position is explained.

When the front cover (5) is located in the retracted position, the front-side engaging protrusions (5a) formed on the inner circumferential surface of the cover (5) are housed in the front-side circumferential-direction locking sections (25) formed on the outer circumferential surface of the cover guide (6) and block the advance of the cover (5) resisting the urging force of the spring (14).

When the front cover (5) is rotated with respect to the hub (4) and the cover guide (6) from this state, the engaging protrusions (5a) relatively move along the locking sections (25) and move to positions overlapping the front-side front-back-direction grooves (24) of the guide (6).

When the grooves (24) move to the positions of the engaging protrusions (5a), the protrusions (5a) are capable of advancing along the grooves (24). The cover (5) advances to the advanced position with the urging force of the spring (14).

When the front cover (5) is located in the advanced position, the engaging protrusions (5a) are located in the blocking sections (26) formed on the distal end side of the grooves (24). The advance and the retraction of the cover (5) are blocked.

Note that, in the embodiments explained above, the front cover (5) is urged by the spring (14) functioning as urging means. However, it is also possible to adopt other components as the urging means.

For example, it is also possible that a member having a bellows shape is adopted as the urging means, when the cover (5) is located in the retracted position, the bellows-shaped member maintains a contracted state and, thereafter, when the cover (5) is located in the advanced position, the cover (5) is advanced using an urging force of the bellows-shaped member returning to an original expanded state.

### Reference Signs List

- 1: needle assembly
- 2: front cannula
- 3: back cannula
- 4: hub
- 5: front cover
- 5a: front-side engaging protrusion
- 6: cover guide
- 7: back cover
- 24: front-side front-back-direction groove
- 25: front-side circumferential-direction locking section
- 33a: back-side engaging protrusion
- 41: coupling section
- 42: back-side front-back-direction groove
- 43: back-side circumferential-direction locking section

## Claims

1. A needle assembly comprising:
a cannula in which a puncture section is formed;
a hub that holds the cannula; and
a cover provided to be movable to a first position where the cannula is exposed with respect to the hub and a second position where a distal end projects further than the cannula, **characterized in that**
in the cover, a circumferential-direction locking section formed in a circumferential direction and a front-back-direction groove formed in a front-back direction to continue to the circumferential-direction locking section or an engaging protrusion movable along the circumferential-direction locking section and the front-back-direction groove are provided,
the movement of the cover in the front-back direction is blocked in a state in which the cover is located in the first position and the engaging protrusion is housed in the circumferential-direction locking section, and
when the engaging protrusion moves along the circumferential-direction locking section and moves to the front-back-direction groove, the engaging protrusion becomes movable along the front-back-direction groove to allow the movement of the cover to the second position.

2. The needle assembly according to claim 1, **characterized in that**
the cannula is a front cannula, at a distal end of which the puncture section is formed, and the cover is a front cover provided to be movable to a retracted position serving as the first position where the cannula is exposed with respect to the hub and an advanced position serving as the second position where the distal end projects further to a front than the cannula,
urging means for urging the front cover toward the advanced position and a cover guide that houses at least a part of the urging means and the front cover are provided to be capable of rotating in the circumferential direction with respect to the hub and the front cover,
the engaging protrusion is provided in one of the cover guide and the front cover, and the circumferential-direction locking section and the front-back-direction groove are provided in the other,
in a state in which the front cover is located in the retracted position, the engaging protrusion engages with the circumferential-direction locking section and blocks advance of the front cover resisting an urging force of the urging means, and
when the front cover is rotated with respect to the cover guide, the engaging protrusion moves from the circumferential-direction locking section to the front-back-direction groove, and the front cover moves to the advanced position with the urging force of the urging means.

3. The needle assembly according to claim 2, **characterized in that** rotation preventing means is provided, the rotation preventing means preventing the front cover and the hub from relatively rotating when the front cover is located in the retracted position, even if the cover guide rotates relatively to the front cover and the hub.

4. The needle assembly according to claim 1, **characterized in that**
the cannula is a front cannula, at a distal end of which the puncture section is formed, and the cover is a front cover provided to be movable to a retracted position serving as the first position where the front cannula is exposed with respect to the hub and an advanced position serving as the second position where the distal end projects further to a front than the front cannula,
Urging means for urging the front cover toward the advanced position is provided, a cylindrical cover guid coupled to the hub is provided on an inside of the front cover, and the front cover is provided to be capable of rotating in the circumferential direction with respect to the hub and the cover guide,
the engaging protrusion is provided in one of the cover guide and the front cover, and the circumferential-direction locking section and the front-back-direction groove are provided in the other,
in a state in which the front cover is located in the retracted position, the engaging protrusion engages with the circumferential-direction locking section and blocks advance of the front cover resisting an urging force of the urging means, and
when the front cover is rotated with respect to the cover guide, the engaging protrusion moves from the circumferential-direction locking section to the front-back-direction groove, and the front cover moves to the advanced position with the urging force of the urging means.

5. The needle assembly according to claim 1, **characterized in that**
the cannula is a back cannula in which a puncture section punctured into a lid section of liquid supplying means is formed at a rear end, and liquid is supplied from the liquid supplying means to the back cannula by piercing the puncture section of the back cannula through the lid section of the liquid supplying means,
the cover is a back cover movable to an advanced position serving as the first position and a retracted position serving as the second position and including a coupling section coupled to a coupled section of the liquid supplying means,
in a state in which the back cover is located in the advanced position, the coupled section of the liquid supplying means is coupled to the coupling section, and the puncture section of the back cannula pierces through the lid section of the liquid supplying means, and
in a state in which the back cover is located in the retracted position, a rear end portion of the back cover projects further to a back than the puncture section of the back cannula and covers the puncture section.

6. The needle assembly according to claim 5, **characterized in that** the back cover has a cylindrical shape and surrounds an outer circumference of the hub.

7. The needle assembly according to claim 5 or 6, **characterized in that** the front-back-direction groove and the circumferential-direction locking section are formed in the back cover, and the engaging protrusion is formed on an outer surface of the hub.

8. The needle assembly according to claim 7, **characterized in that** a housing section that houses the engaging protrusion is formed in a front of the front-back-direction groove and the housing section is formed to pierce through the back cover from an inside to an outside to enable the engaging protrusion to be visually recognized from an outside when the engaging protrusion is housed in the housing section.
